# EUROPÄISCHE PATENTSCHRIFT

(11) **EP 0 914 151 B1**
(45) Veröffentlichungstag und Bekanntmachung des Hinweises auf die Patenterteilung: **10.10.2001**
(21) Anmeldenummer: 97930477.1
(22) Anmeldetag: 02.07.1997
(51) Int. Cl.: A61K 39/00, A61K 39/39, A61K 39/12, A61K 39/21, A61K 31/70, A61K 39/395, A61K 35/12

(54) **KOMPLEX AUS ANTIGEN UND ZELLBESTANDTEIL, VERFAHREN ZUR DESSEN HERSTELLUNG UND THERAPEUTISCHE VERWENDUNGEN**
COMPLEX COMPRISING ANTIGEN AND CELL CONSTITUENT, METHOD OF PREPARING SAID COMPLEX AND ITS THERAPEUTIC USES
COMPLEXE FORME D'UN ANTIGENE ET D'UN CONSTITUANT CELLULAIRE, PROCEDE DE PRODUCTION DUDIT COMPLEXE ET SES UTILISATIONS THERAPEUTIQUES

(30) Priorität: 02.07.1996 DE 19626614
(43) Veröffentlichungstag der Anmeldung: 12.05.1999
(73) Patentinhaber: Bio-Products & Bio-Engineering Aktiengesellschaft, 1010 Wien (AT)
(72) Erfinder: EIBL, Martha, A-1180 Wien (AT); KREIL, Thomas, A-3400 Klosterneuburg (AT); MANNHALTER, Josef, A-1050 Wien (AT); EIBL, Johann, A-1180 Wien (AT); KERSCHBAUM, Astrid, A-1180 Wien (AT); BRÜHL, Peter, A-1150 Wien (AT)
(74) Vertreter: Schwarz, Albin, Dr.
(86) Internationale Anmeldenummer: EP9703452
(87) Internationale Veröffentlichungsnummer: WO9800162

(56) Entgegenhaltungen:
- EP-A- 0 284 791
- WO-A-95/24924
- M. HEIKE ET AL.: "Membranes activate tumor- and virus-specific precursor cytotoxic T lymphocytes in vivo and stimulate tumor-specific T lymphocytes in vitro: Implications for vaccination." JOURNAL OF IMMUNOTHERAPY, Bd. 15, Nr. 3, April 1994, NEW YORK, NY, VSA, Seiten 165-174, XP002048220 in der Anmeldung erwähnt
- J. WARNER ET AL.: "Induction of HIV-specific immune responses using retroviral vectors." INTERNATIONAL CONFERENCE ON AIDS, Bd. 8, Nr. 2, 19. - 24.Juli 1992, AMSTERDAM, NL, Seite B164 XP002048221
- J. WARNER ET AL.: "Induction of HIV-specific CTL and antibody responses in mice using retroviral vector-transduced cells." AIDS RESEARCH AND HUMAN RETROVIRUSES, Bd. 7, Nr. 8, August 1991, NEW YORK, NY, VSA, Seiten 645-655, XP002048222
- L. MYLIN ET AL.: "Cytotoxic T lymphocyte escape variants, induced mutations, and synthetic peptides define a dominant H-2Kb-restricted determinant in simian virus 40 tumor antigen." VIROLOGY, Bd. 208, Nr. 1, 1.April 1995, SAN DIEGO, CA, VSA, Seiten 159-172, XP002048223
- Y. BECKER: "Dengue fever virus and Japanese encephalitis virus synthetic peptides, with motifs to fit HLA class I haplotypes prevalent in human populations in endemic regions, can be used for application to skin Langerhans cells to prime antiviral CD8+ cytotoxic T cells (CTLs)." VIRUS GENES, Bd. 9, Nr. 1, September 1994, BOSTON, MA, VSA, Seiten 33-45, XP002048224
- K. VENUGOPAL ET AL.: "Towards a new generation of flavivirus vaccines." VACCINE, Bd. 12, Nr. 11, August 1994, OXFORD, GB, Seiten 966-975, XP002048225

## Beschreibung

Die vorliegende Erfindung betrifft einen Komplex von einem Antigen eines Pathogens mit einem Zellbestandteil. Im weiteren wird eine Impfstoffzusammensetzung, welche den Komplex enthält, eine entsprechende pharmazeutische Präparation sowie ein Verfahren zur Herstellung des Komplexes zur Verfügung gestellt.

Mikroben, wie Viren, Bakterien, Pilze oder Parasiten, können eine Vielzahl von Wirtsorganismen infizieren, pathologische Schädigungen hervorrufen und im Falle einer unkontrollierten Vermehrung ihren Wirt auch töten. Dem Immunsystem ist es zu verdanken, dass Erreger erkannt und effizient bekämpft werden.

In den letzten Jahren werden Prionen als neue infektiöse Pathogene der molekularen Art diskutiert. Prusiner S. et al., Advances in Virus Research, Vol. 29, pp 1-56, 1984, beschreiben die Krankheiten, die auf Prionen zurückgeführt werden, darunter Scrapie, Kuru, Creutzfeldt-Jakob Erkrankung , Gerstmann-Sträussler-Schenker Syndrom und familiäre Insomnia. Obwohl den Prionen eine molekulare Struktur zugeordnet wird, können entsprechende Nukleinsäuren, die für die Expression der Prionen verantwortlich sind, nicht ausgeschlossen werden. Die Kultivierung von Prionen *in vitro* bzw. die Infektion von Zell-Linien zur Gewinnung eines entsprechenden Titers wird von Race R. et al., Current Topics in Microbiology and Immunology, Vol. 172, pp 181-193, beschrieben.

Die Behandlung von Patienten, die dem Risiko einer Infektion aufgrund von Prionen bzw. "slow viruses" ausgesetzt sind, gewinnt zunehmend an Bedeutung. Die dafür zur Verfügung stehenden Tiermodelle und isolierten Moleküle können zur Testung von entsprechenden Behandlungsstrategien herangezogen werden.

Funktionell besteht das Immunsystem aus angeborenen, relativ unspezifischen Resistenzmechanismen einerseits, und erworbenen, sehr spezifischen Effektorfunktionen der Immunität. Bei den Effektormechanismen der Immunität ist weiterhin zwischen einer humoralen Immunantwort, also der Bildung von Antikörpern gegen Antigene eines Pathogens, und der zellulären Immunantwort zu unterscheiden.

Die Immunität gegen ein infektiöses Pathogen kann durch aktive oder passive Immunisierung erzeugt werden. Bei der passiven Immunisierung wird der humorale bzw. zelluläre Schutz von einem Organismus, der diesen Schutz aufgebaut hat, auf einen anderen, naiven Organismus übertragen. Die Wirkung tritt sofort ein, die Dauer der Protektion ist allerdings beschränkt.

Aktive Immunität kann durch eine Infektion mit dem Pathogen induziert werden, andererseits kann sie aber auch durch Verabreichung eines Impfstoffes erzielt werden. Das Immunsystem wird bei diesen beiden Vorgängen aktiviert, was beispielsweise zur Proliferation von Antigen-reaktiven T- oder B-Zellen führt, aus denen in weiterer Folge Gedächtniszellen entstehen. Die aktive Immunität bleibt daher über einen langen Zeitraum, auch jahrelang erhalten und kann, wenn nötig, durch eine Nachimpfung wieder aufgefrischt werden.

Für die aktive Immunisierung stehen zahlreiche Impfstoffe gegen eine Vielzahl infektiöser Pathogene zur Verfügung, und genauso vielfältig ist das Design solcher Vakzinen.

Folgende Arten von Impfstoffen sind derzeit vorwiegend in Verwendung: Ganzvirusvakzinen (als inaktivierte, abgetötete Vakzinen oder als attenuierte Lebendviren), gereinigte oder rekombinant hergestellte Vakzinen, bestehend aus Untereinheiten des Pathogens, sogenannte subunit Vakzinen, rekombinante Vektor-Vakzinen, synthetische Peptid-Vakzinen oder anti-idiotypische Vakzinen. Neuere Entwicklungen bei der Herstellung von Impfstoffen gehen z.B. in Richtung Verwendung "nackter" Nukleinsäuren (z.B. DNA Vakzinen).

Je nach Design der Vakzine und Form der Verabreichung ist eine unterschiedliche Art bzw. ein unterschiedliches Ausmass der entsprechenden Immunreaktion zu erwarten. Ein häufig auftretendes Problem bei der Vakzinierung ist die geringe Immunogenität der verabreichten Vakzine bzw. das Problem, dass auch eine mehrfache Verabreichung zu keiner protektiven Immunität führt.

Aus diesen Gründen werden den Impfstoffen häufig sogenannte Immunstimulantien oder Adjuvantien zugesetzt. Solche Stoffe bewirken eine Verstärkung der Immunantwort (Immunpotentatoren), oder sie beeinflussen die Art der Immunantwort (Immunmodulatoren). Im Stand der Technik sind hierfür beispielsweise anorganische Stoffe, wie beispielsweise Aluminiumhydroxid, seit langem bekannt, ebenso wie die Verwendung von Wasser-in-Öl Emulsionen mit (komplettes Freund'sches Adjuvans) und ohne Mykobakterien (inkomplettes Freund'sches Adjuvans).

Auch verschiedene Zytokine, wie z.B. Interleukine oder Lymphokine, besitzen adjuvantierende Eigenschaften.

Bei der Wahl eines Adjuvans bzw. einer entsprechenden Kombination aus Antigen und Adjuvans ist zu beachten, dass einerseits nicht jedes Antigen durch ein beliebiges Adjuvans eine verstärkte Immunantwort erhält, und dass andererseits auch die Art der induzierten Immunantwort von dem verwendeten Adjuvans beeinflusst werden kann. Deshalb ist es nach wie vor von Interesse, Stoffe mit adjuvantierenden Eigenschaften für Vakzinen zu finden.

Aus einer neueren Arbeit (Heike M. et al., 1994, J.Immunotherapy 15: 165-174) ist beispielsweise die *in vitro* Stimulation von zytotoxischen T-Zellen durch Plasmamembranen von Mäuse-Fibroblasten bekannt. In dem Dokument wird auch die Induktion antigen-spezifischer, MHC-Klasse I restringierter zytotoxischer T-Zellen beschrieben, die nach Immunisierung von Mäusen mit syngenen Membranen von UV-induzierten Tumorzellen, SV40 transformierten Fibroblasten oder nicht-produktiv mit Influenza infizierten Fibroblasten, festgestellt worden ist. Diese Membranen werden an Mäuse verabreicht um eine zelluläre Immunantwort hervorzurufen.

Auch aus Bachmann M.F. et al., 1994, Eur.J.Immunology 24, 2128-2236 ist bekannt, dass zelluläre Fragmente von Insektenzellen, als Adjuvans für ein rekombinantes Antigen zur Induktion zytotoxischer T-Zellen eingesetzt werden können. Das Antigen und die Membran oder das Membranfragment kann als ein Gemisch verabreicht werden.

Neben der Induktion einer zellulären Immunität ist meist auch die Induktion einer humoralen Immunantwort wünschenswert, da für verschiedene Pathogene die Bedeutung von humoraler und zellulärer Immunität zwar unterschiedlich gross ist, meist aber beide zum Erfolg einer Immunantwort beitragen.

Seit Peiris J.S. and Porterfield J.S., 1979, Nature 282, 509-511, ist allerdings auch bekannt, dass Antikörper gegen ein Viruspartikel mit einer effizienteren Infektion des Virus in Verbindung gebracht werden können ("antibody dependent enhancement of viral infectivity"). Antikörper gegen ein Virus können daher auch zu einer Verschlechterung der klinischen Prognose beitragen, und ein Problem dieser Art versucht man bei der Entwicklung eines Impfstoffes gerade zu vermeiden.

Ein weiteres Problem, welches gerade bei Verwendung sogenannter "subunit"-Vakzinen auftreten kann, ist eine möglicherweise zu geringe Immunogenität des verabreichten Antigens, die im Zusammenhang mit einer nicht ausreichenden Präsentation des Antigens für immunkompetente Zellen stehen kann.

Eine Aufgabe der vorliegenden Erfindung ist es daher, ein hochwirksames Immunogen unter Vermeidung der aus dem Stand der Technik bekannten Nachteile zur Verfügung zu stellen. Unter anderem soll erreicht werden, dass die antigene Determinante so präsentiert wird, dass eine protektive Immunität erzielt werden kann, dass also bei einer Infektion mit dem Pathogen diesem gegenüber ein ausreichender Schutz gegeben ist.

Diese Aufgabe wird erfindungsgemäss durch den Gegenstand der vorliegenden Patentansprüche gelöst.

Gegenstand des Patentanspruchs 1 ist somit ein Antigen eines mikrobiellen oder molekularen Pathogens das mit einem Zellbestandteil komplexiert ist in isolierter bzw. gereinigter Form, wobei der Zellbestandteil von Säugetierzellen stammt. Dabei sind jene Säugetierzellen bevorzugt, die von dem Pathogen infizierbar sind bzw. die das Antigen exprimieren.

Überraschenderweise wurde gefunden, dass der erfindungsgemässe Komplex eine ausgezeichnete Fähigkeit zur Induktion einer protektiven Immunität, ausgedrückt durch eine humorale und gegebenenfalls eine zelluläre Immunantwort, besitzt.

Gerade durch die Assoziation des Antigens mit dem Zellbestandteil kann nämlich in besonderen Fällen einerseits das entsprechende Antigen sehr gut präsentiert werden, andererseits kommen, bei Verabreichung des Komplexes, beispielsweise an einen Säuger, auch co-stimulatorische Signale aufgrund des Zellbestandteils zur Wirkung.

Der Zellbestandteil des erfindungsgemässen Komplexes stammt von einer tierischen oder humanen Zelle, oder ist ein synthetisches Äquivalent. Damit werden in einem entsprechenden Präparat, das an einen Menschen verabreicht wird, Bestandteile von Prokaryonten oder Insekten vermieden.

Als Ausgangsmaterial für den Zellbestandteil des erfindungsgemässen Komplexes werden beispielsweise autologe, homologe oder heterologe Zellen verwendet. Dabei werden insbesondere solche mit der Fähigkeit zur kompetenten Antigen-Präsentation bevorzugt ausgewählt. Auch Erythrozyten, Leukozyten, Thrombozyten, Lymphozyten, Thymozyten, Granulozyten, Monozyten, primäre Zellen oder Zell-Linien können als bzw. zur Produktion des Zellbestandteiles verwendet werden.

Beispiele für Säugerzell-Linien sind für die Impfstoffproduktion geeignete Zell-Linien wie VERO Zellen, CHO oder HeLa Zellen in Frage, aber auch maligne bzw. immortalisierte Zellen, speziell Tumorzellen, eignen sich für den erfindungsgemässen Einsatz.

Die verwendeten Zellbestandteile haben vorzugsweise co-stimulatorische Wirkung und ermöglichen beispielsweise eine Aktivierung von Zellen. In einer bevorzugten Ausführungsform sind die Zellbestandteile von einer stimulierten bzw. aktivierten Zelle abgeleitet. So eine Zelle ist dadurch erhältlich, dass man sie besonderen Reizen aussetzt. Beispielsweise werden den Zellen Mitogene, Hormone, Zytokine oder andere Zellen zugesetzt, um eine Reaktion bzw. Veränderung der Zelle zu provozieren. Vorzugsweise sind die Zellbestandteile nicht von ruhenden Zellen, insbesondere nicht von ruhenden Fibroblasten.

Der Zellbestandteil des erfindungsgemässen Komplexes ist vorzugsweise von infizierten, insbesondere mit dem Pathogen infizierten Zellen abgeleitet. Nach einer bevorzugten Ausführungsform ist der Zellbestandteil von einer Zelle abgeleitet, die Vollvirus produziert oder von einer solchen Zelle, die virale Bestandteile repliziert. Es handelt sich also um keine latent infizierte Zelle.

In einer bevorzugten Ausführungsform ist der Zellbestandteil des erfindungsgemässen Komplexes unmittelbar aus der mit dem Pathogen infizierten Zelle erhältlich. Hierfür wird die Zelle, vorzugsweise eine für die Produktion von Humanvakzinen geeignete Zell-Linie nach herkömmlichen Methoden mit dem Pathogen infiziert, beispielsweise mit einem Virus, am meisten bevorzugt mit einem humanpathogenen Virus. Danach wird ein Zellbestandteil gewonnen, beispielsweise eine Membranfraktion der virus-infizierten Zelle, an der dann Antigene des Pathogens gebunden sind bzw. werden, die im ungebundenen, reifen Virus an sich nicht vorliegen. Im speziellen handelt es sich dabei um sogenannte nicht-strukturelle Proteine eines Virus.

Bevorzugterweise wird der Zellbestandteil in einer Fraktion erhalten, die die Zellmembran bzw. eine intrazelluläre Struktur der Zelle enthält. Der Zellbestandteil kann aber auch die Zelle als solche darstellen. Im Fall einer Membran oder eines Membranfragmentes als Zellbestandteil kann es sich um eine Plasmamembran oder um eine Membran von Zellorganellen handeln. Als Zellorganellen sind hier zu nennen: das endoplasmatische Retikulum, der Golgi Apparat, Lysosomen und weitere.

Der Zellbestandteil kann erfindungsgemäss auch ein gegebenenfalls synthetisches Äquivalent einer Membran bzw. eines Membranfragmentes darstellen mit einer ähnlichen Struktur und gleichen Funktion wie in dem erfindungsgemässen Komplex. Dieser Bestandteil oder dessen entsprechendes Äquivalent ist nach einer weiteren Ausführungsform auch synthetisch hergestellt, beispielsweise mittels chemischer Methoden.

Die Proteinanteile der Membran können auch rekombinant hergestellt werden und mit Lipiden zu einer Membran assembliert werden, bzw. sind auch synthetisch hergestellte Liposomen als Membranen einsetzbar. Gleichfalls können auch Mischungen verschiedener synthetisch hergestellter oder natürlich vorkommender Lipide bzw. Phospholipide enthalten sein.

Im weiteren eignet sich auch der Einsatz von Mischungen der vorstehend spezifizierten Membranen, Membranfragmente bzw. Zellstrukturen zur Herstellung des erfindungsgemässen Komplexes.

Der Zellbestandteil ist in einer weiteren bevorzugten Ausführungsform von Zellen, die von der Spezies stammen, für die das Virus pathogen wäre, abgeleitet. Beispielsweise ist das Virus ein human-pathogenes Virus und der Zellbestandteil ist von einer humanen Zelle abgeleitet.

In einer weiteren bevorzugten Ausführungsform ist der Zellbestandteil eine Substanz ausgewählt aus der Gruppe Membranprotein, intrazelluläres Protein, von aktivierten Lymphozyten abgeleiteter Faktor und Aktivator für Lymphozyten oder eine Kombination davon bzw. enthält die Zellfraktion eine derartige Substanz oder Kombination von Substanzen. Eine derartige Substanz ist erhältlich aus einer Zellfraktion oder wird als gereinigte Substanz eingesetzt. Das Membranprotein oder das intrazelluläre Protein ist vorzugsweise ein Alloantigen, Chemokin-Rezeptor, Chemokin oder Lymphokin. Auch alle Kombinationen davon sind erfindungsgemäß möglich. Das Alloantigen ist insbesondere ein MHC-Klasse I oder MHC-Klasse II Antigen. Als intrazelluläres Protein soll beispielsweise das Hitzeschockprotein genannt werden. Ein von aktivierten Lymphozyten abgeleiteter Faktor ist beispielsweise ein solcher Faktor, der die Fähigkeit besitzt das Viruswachstum zu unterdrücken. Unter Aktivator für Lymphozyten ist erfindungsgemäß eine Substanz zu verstehen, die beispielsweise einen vorhin erwähnten Faktor induzieren kann.

Der Zellbestandteil kann beispielsweise nach gezielter Transformation, Transfektion oder Aktivierung einer entsprechenden Zelle erhalten werden. Die vorhin genannten Substanzen können den Zellen aber auch als Zusätze in definierten Mengen beigemengt werden.

In einer weiteren bevorzugten Ausführungsform ist der Zellbestandteil von einer ein rekombinantes Gen enthaltenden Zelle abgeleitet. Damit wird die Verwendung eines Antigens, welches von einer Nukleinsäure des Pathogens kodiert wird, die in eine Zelle eingeschleust und von der Zelle exprimiert wird möglich. Es werden zu diesem Zwecke gentechnologische Methoden bzw. rekombinante Nukleinsäuren, also beispielsweise rekombinante DNA-Technologien, verwendet.

In einer derartigen Wirtszelle kommt es zur Expression von Antigenen, welche von dem Genom des Pathogens kodiert werden. Die Nukleinsäure des Pathogens bzw. Teile davon können jedoch auch über einen geeigneten Vektor, wie rekombinante Viren, bzw. Plasmide oder direkt in das Genom der Wirtszelle eingebracht werden, sodass die transformierte bzw. transfizierte Zelle die gewünschten Antigene des Pathogens exprimiert.

Für diese Technologien stehen eine Reihe von Methoden zur Verfügung. Die Konstruktion entsprechender Vektoren beinhaltet vorzugsweise geeignete Promotorelemente wie z.B. SV40-, CMV-, RSV-, LTR-, EBV- β-Actin-, hGH-, T4-, T7-, SP6-, Metallothionein-, Adeno-2, "Adeno Major Late-" oder TK Promotor oder muskelspezifische Promotoren wie Myosinpromotoren oder induzierbare Promotoren wie hsp- oder β-Interferonpromotoren. Beispiele für entsprechende DNA-Expressionsvektorsysteme beinhalten pBPV, pSVL, pRc/CMV, pRc/RSV, myogenische Vektorsysteme (WO 93/09236) oder Vektoren von viralen Systemen wie Poxviren (US 5,445,953), Adenoviren, Retroviren, Baculoviren, Herpesviren und Polioviren.

Der Expressionsvektor kodierend für das Antigen wird dann zum Transformieren der Wirtszelle verwendet. Die Wirtszelle ist eine eukaryontische Wirtszelle. Bevorzugte Säugerzellen sind CHO, COS, BHK, SK-HEP, C127, MRC5, 293, Verozellen, Fibroblasten, Keratinocyten oder Myoblasten, Hepatocyten oder Stammzellen.

Das entsprechende Antigen ist vorzugsweise ein protektives Antigen oder es wirkt durch die Assoziation an den Zellbestandteil protektiv. Der Begriff Protektion bedeutet, dass ein Schutz gegen eine Infektionskrankheit gegeben ist, wobei dieser Schutz nachgewiesen werden kann durch ein entsprechendes Tiermodell bzw. mit Hilfe eines eindeutig erwiesenen Surrogatmarkers, beispielsweise mit einem bestimmten Antikörpertiter.

Als protektive Antigene des Pathogens gelten insbesondere Peptide, Polypeptide bzw. Proteine abgeleitet von dem Genom eines Pathogens, für welche im erfindungsgemässen Komplex die protektive Wirkung nachgewiesen werden kann; es kann aber auch eine entsprechende Nukleinsäure des Pathogens eingesetzt werden. Vorzugsweise ist das Pathogen ein Virus, am meisten bevorzugt ein Virus ausgewählt aus der Gruppe bestehend aus Flaviviren, Herpesviren, Hepatitis-Viren, Retroviren, Influenza-Viren, Cocksackievirus, Tumorviren und Echoviren.

Ausgewählte Viren sind aus der Familie der Flaviviridae, vorzugsweise Enzephalitits Virus, wie TBE-Virus, insbesondere TBE-Virus des westlichen Subtyps (FSME-Virus), oder ausgewählt aus der Subgruppe der Dengue Viren. Weitere ausgewählte Viren sind aus der Familie der Hepadnaviridae, vorzugsweise das Hepatitis A, B, C, D oder X Virus. Ebenso ausgewählte Viren sind aus der Familie der Orthomyxoviridae, vorzugsweise Influenza Viren. Aus der Familie der Picornaviridae werden vorzugsweise Viren aus dem Genus der Enteroviren ausgewählt, beispielsweise Coxsackievirus, oder aus dem der Rhinoviren. Aus der Familie der Retroviridae wird vorzugsweise ein HI-Virus, insbesondere HIV-1 und HIV-2, ausgewählt.

Antigene von bakteriellen Pathogenen sind vorzugsweise abgeleitet von E. coli, Bordetella, Borrelia, Pseudomonas, Haemophilus, Mycobakterien, Streptokokken, Salmonellen, Helicobacter und Clostridien. Die Antigene, welche von Parasiten stammen, sind vorzugsweise ausgewählt aus der Gruppe bestehend aus Amaebida, Trypanosoma und Plasmodium. Antigene die von einem molekularen Pathogen stammen, sind vorzugsweise von den Erregern der Creutzfeldt-Jakob Krankheit, Scrapie, Kuru und BSE abgeleitet.

Wird der erfindungsgemässe Komplex in einer bevorzugten Ausführungsform durch Infektion einer Zelle mit einem Virus erhalten, so kann das Antigen bzw. der Komplex in einer frühen Phase der Virus-Replikation erhalten werden. Daneben ist es aber auch möglich, das Antigen bzw. den Komplex in der späten Phase der Virus-Replikation zu erhalten. Je nach dem Replikationsstadium des Virus ist es möglich, bestimmte Antigene bzw. Zellbestandteile zu erhalten, die in der ruhenden Zelle bzw. im Vollvirus nicht oder nicht in dieser Form vorhanden sind.

Ein solches Beispiel für ein Protein mit verschiedenen Erscheinungsformen während der Replikation eines Virus ist das preM Protein, welches beim Aufbau eines Flaviviridae-Virus auftritt und im reifen Viruspartikel nicht mehr vorhanden ist, da es vollständig zum M Protein prozessiert worden ist. Beispiel für ein Virus-kodiertes Protein, das im freien Virus-Partikel nicht vorkommt, ist etwa NS1, NS3, NS5 oder andere nicht-strukturelle Proteine der Flaviviridae.

Im erfindungsgemässen Komplex kann das Antigen mehrere Epitope ausbilden und sogar ein Ganzpathogen, z.B. ein Ganzvirus insbesondere ein inaktiviertes oder attenuiertes Virus, oder ein Teil des Pathogens, z.B. ein subvirales Partikel, darstellen.

In einer bevorzugten Ausführungsform umfasst der erfindungsgemässe Komplex eine Zusammensetzung von Antigenen, die für die Protektivität verantwortlich ist, insbesondere wenigstens ein Nichtstruktur-Protein des Pathogens. Bei dem Nichtstruktur-Protein handelt es sich beispielsweise um ein regulatorisches Protein, ein Protein mit enzymatischer Funktion wie eine Reverse Transkriptase, RNA-Polymerase, Integrase oder Protease, oder ein Protein ohne bzw. mit einer derzeit noch nicht bekannten Funktion. Es werden auch entsprechende Vorläufermoleküle dieser Proteine oder strukturelle Proteine sowie entsprechende Derivate mit einer vergleichbaren oder sogar verbesserten immunogenen Wirkung, wie z.B. (chemisch) modifizierte Proteine, Fragmente, Fusionsproteine, Mutanten, Analoge und dgl. erfindungsgemäss eingesetzt. Die Mutante haben bevorzugterweise eine Aminosäuresequenz mit mindestens 80% Homologie zu dem entsprechenden natürlichen Protein.

Für den Fall, dass der erfindungsgemässe Komplex ein nicht-strukturelles Protein eines Pathogens umfasst, bietet sich die Möglichkeit, dass bei dessen Verwendung als Vakzine eine Immunisierung mit Antigenen erreicht werden kann, die bei Verwendung üblicher "subunit"- Vakzinen oder Vakzinen auf Basis von Ganzpathogenen nicht erreicht werden kann. Dies ist insbesondere in jenen Systemen von Vorteil, wo durch Antikörper gegen Strukturproteine eines Virus ein "antibody-dependent enhancement of infection" zu befürchten ist. Eine Immunität gegen eine nicht-strukturelle Komponente des Virus ermöglicht eine erfolgreiche Immunantwort ohne diesen Nachteil.

Die weiters bevorzugten Antigene bestehen aus mehreren Untereinheiten (subunits). Beispielsweise bestehen diese komplexen Antigene aus einem Hüllprotein und/oder einem Kernprotein des Pathogens und einer nicht-strukturellen Komponente des Pathogens. Vorzugsweise liegt wenigstens ein Nichtstrukturprotein und ein Strukturprotein als komplexes Antigen vor.

Die Komplexbindung in dem erfindungsgemässen Komplex ist beispielsweise eine kovalente Bindung, gegebenenfalls durch ein Bindeglied ("linker"). Eine kovalente Bindung ist aber auch durch chemische Vernetzung möglich. Die einfache Ausbildung von Schwefelbrücken ist ebenfalls eine Art einer kovalenten Bindung. Die Komplexbindung kann aber auch aufgrund von elektrostatischen, hydrophoben oder van der Waals schen Kräften bestehen. In einer weiteren bevorzugten Ausführungsform ist das Antigen und der Zellbestandteil an einem festen Träger adsorbiert bzw. co-adsorbiert. Der feste Träger kann beispielsweise auch eine Lipidkomponente, wie ein Liposom oder Phospholipidvesikel, sein.

Die Bindung des Antigens an den Zellbestandteil im erfindungsgemässen Komplex bleibt überraschenderweise auch bestehen wenn eine der beiden Komponenten oder beide weiter behandelt werden. Beispielsweise kann eine Behandlung zur Inaktivierung vorgenommen werden, beispielsweise Beschallung, Bestrahlung, eine chemische Behandlung, Hitzebehandlung oder Enzymbehandlung. Vorzugsweise wird eine chemische Behandlung mit Formalin durchgeführt, so dass das Pathogen nicht mehr infektiös ist bzw. nach Verabreichung nicht mehr pathogen wirkt.

Die Zelle kann erfindungsgemäss unbehandelt oder in modifizierter Form als Ausgangsmaterial zur Herstellung des erfindungsgemässen Komplexes verwendet werden. Sie kann einer Lyse unterworfen werden oder als intakte Zelle vorliegen. Zur Präparation einer Zellfraktion werden die Zellen beispielsweise lysiert, das Zell-Lysat fraktioniert, gegebenenfalls durch eine Dichtegradienten-Zentrifugation, und die entsprechende Fraktion isoliert.

In einer weiteren bevorzugten Ausführungsform liegt der erfindungsgemässe Komplex in gereinigter Form vor. Diese Reinigung kann mittels verschiedener Methoden zur Reinigung von hochmolekularen Substanzen erfolgen. Eine solche Reinigung kann beispielsweise nach Grösse oder nach Ladung des Stoffes erfolgen. Beispielsweise wird ein erfindungsgemässer immunogener Komplex durch Zentrifugation (z.B. Dichtegradientenzentrifugation), Dialyse/Diafiltration, chemische Fällung oder chromatographisch, insbesondere durch Gelfiltration, Ionenaustausch, hydrophobe Chromatographie, Affinitätschromatographie oder "reversed phase" Chromatographie gereinigt. Auch eine Kombination von Reinigungsverfahren ist zur Erhaltung eines hochgereinigten Immunogens möglich. Durch die Reinigung werden freie Antigene, also Antigene, die nicht an Zellbestandteile assoziiert sind, abgetrennt. Bevorzugterweise werden vorhandene Zellkerne entfernt. Ebenso werden auch freie Zellbestandteile abgetrennt. Der erfindungsgemässe Komplex ist in seiner gereinigten Form insoweit charakterisiert, dass eine weitere Reinigung zu keiner wesentlichen Abtrennung von freiem Antigen bzw. Zellbestandteil führt.

Antigen und Zellbestandteil können auch unabhängig voneinander gereinigt werden, und die Assoziation zum Komplex erfolgt dann im Anschluss an die Reinigung oder währenddessen.

Der erfindungsgemässe Komplex kann als ein Präparat vorliegen, welches zusätzlich einen Zellbestandteil, wie eine Membran, vorzugsweise abgeleitet von nicht-infizierten, nicht-transfizierten oder nicht-transformierten Zellen, in einer die Immunogenität verstärkenden Menge, also als Adjuvans, enthält. Dieses Präparat eignet sich vor allem zur Immunisierung von Säugern, insbesondere Primaten bzw. Menschen.

Gemäß einer bevorzugten Ausführungsform enthält der erfindungsgemäße Komplex als Antigen ein inaktives Virus. Das Virus ist bevorzugterweise ein inaktiviertes Virus und insbesondere ein durch ein chemisches und/oder physikalisches Verfahren inaktiviertes Virus. Als ein derartiges chemisches Verfahren wird beispielsweise eine Behandlung mit Formaldehyd angewandt, ein physikalisches Verfahren zur Inaktivierung kann beispielsweise in einer Hitze-, Bestrahlungs- bzw. Ultraschallbehandlung bestehen.

In einer besonders bevorzugten Ausführungsform besteht der erfindungsgemäße Komplex aus einem inaktiven Virus, welches einen Zellbestandteil inkorporiert hat, wie dies beispielsweise beim sogenannten budding Prozeß des Virus der Fall ist. Insbesondere hat es einen Zellbestandteil inkorporiert, der Substanzen ausgewählt aus der Gruppe Membranprotein, intrazelluläres Protein, von aktivierten Lymphozyten abgeleiteter Faktor und Aktivator für Lymphozyten enthält bzw. mit derartigen Substanzen angereichert ist.

Gemäß einer bevorzugten Ausführungsform ist die Säugetierzelle bzw. die Zellfraktion an wenigstens eine Bestandteil gemäß einem der Ansprüche 9, 14 oder 15 angereichert.

Diese Anreicherung kann beispielsweise durch Beimengen des gewünschten Bestandteils zur Zelle bzw. Zellfraktion erfolgen, durch Induktion der Bildung der gewünschten Substanz, beispielsweise durch Zugabe von Chemokinen oder Interferonen, z.B. Interferon-gamma, oder durch eine molekularbiologische Methode des Gentransfers, wie z.B. retroviraler Gentransfer oder Transfektion der Zelle mit einem entsprechenden Gen.

Das Pathogen, mit dem die Zelle infiziert wird, kann, wie bereits an früherer Stelle erwähnt, ein Virus sein, insbesondere ein inaktives Virus. Im Falle, daß die Zelle bzw. die Zellfraktion an den beschriebenen Bestandteilen angereichtert ist, kann dann ein Komplex, bestehend aus einem an diesen Bestandteilen angereicherten Virus, erhalten werden.

Erfindungsgemäss wird auch eine Impfstoffzusammensetzung zur Verfügung gestellt, welche den erfindungsgemässen Komplex in einer pharmazeutisch akzeptablen Form und gegebenenfalls weitere Antigene bzw. Adjuvantien enthält. Als zusätzliche Adjuvantien kommen hierfür gängige, aus dem Stand der Technik bekannte Substanzen zum Einsatz. Beispielsweise können verschiedene Zytokine verwendet werden. Im weiteren kommen auch anorganische Substanzen, wie Aluminium- oder Eisenverbindungen, darunter die entsprechenden Hydroxide, zum Einsatz.

Die erfindungsgemässe Impfstoffzusammensetzung liegt vorzugsweise in einer Menge vor, die geeignet ist für die therapeutische oder prophylaktische Behandlung von Säugern, insbesondere Primaten, darunter der Mensch, die das Risiko der Erkrankung aufweisen, welche durch das Pathogen hervorgerufen wird.

Die erfindungsgemässe Impfstoffzusammensetzung ist beipielsweise zur Behandlung von Infektionen bzw. Infektionskrankheiten der HI-Viren, wie AIDS, verschiedener Erkrankungen des Nervenssystems, z.B. Enzephalitis, zur Behandlung von Infektionen mit Influenza Viren, Autoimmunerkrankungen und weiteren geeignet.

Ein Vorteil des erfindungsgemässen Komplexes liegt darin, dass das komplexgebundene Antigen das Problem des "enhancement" einer Infektion vermeidet. Die Immunisierung unter Verwendung des erfindungsgemässen Komplexes kann nämlich zu einer Immunität speziell gegen die infizierten Zellen und weniger gegen das freie Pathogen führen. Bei geeigneter Wahl des Zellbestandteiles und einer nicht-strukturellen Komponente des Pathogens wird also speziell eine Immunität gegen diese nicht-strukturelle Komponente des Pathogens induziert, die gegen die mit dem Pathogen infizierte Zelle gerichtet ist, nicht aber zu einer "antibody-dependent enhancement" der Infektion führt.

Im weiteren kann die Wirkung der erfindungsgemässen Vakzine universell sein und gegen mehrere Subtypen eines Virus, beispielsweise die Dengue Viren, oder mehrere Mitglieder derselben Virusfamilie, beispielsweise mehrere Flaviviren, gerichtet sein, z.B. durch Induktion einer Immunität gegen konservierte, nicht-strukturelle Proteine, speziell solche mit regulatorischer Funktion.

Die erfindungsgemässe Impfstoffzusammensetzung kann in einer für die parenterale oder für die Stimulation der Mukosaimmunität geeigneten Form vorliegen. Beispielsweise liegt sie in einer für die intravenöse, intramuskuläre, transdermale, subkutane oder intraarterielle Verabreichung geeigneten Form vor, oder sie liegt in einer für die mukosale, insbesondere orale, intranasale oder rektale Verabreichung geeigneten Form vor.

Die erfindungsgemässe Impfstoffzusammensetzung kann in Lösung, als Suspension oder feste Präparation, z.B. als Tablette oder als Suppositorium, in einer Fertigspritze oder als Spray zur Verfügung gestellt werden.

Die Kriterien für die Eignung des erfindungsgemässen Komplexes als Vakzine können unterschiedlich sein. Beispielsweise kann die Immunogenität des Komplexes als ein Auswahlkriterium herangezogen werden. Nach Verabreichung an ein Tier kann dann in dem Modell eine entsprechende Immunreaktion, beispielsweise der Antikörpertiter, gemessen werden und die benötigte Menge bzw. Dosis der Vakzine abgeschätzt werden.

Gegebenenfalls kann auch die Protektion als ein weiteres Kriterium für die Eignung des erfindungsgemässen Komplexes zur Herstellung einer Vakzine herangezogen werden. Dies ist beispielsweise dann der Fall, wenn ein "challenge"-Modell für das entsprechende Pathogen zur Verfügung steht. Nach erfolgter Exposition eines Modelltieres mit dem Pathogen kann der Schutz vor dem Ausbruch der Krankheit oder deren Folgen beurteilt werden. Als Massstab zählt beispielsweise die Veränderung von physiologischen Indikatoren, wie Antikörpertiter oder andere Blutwerte, bzw. der Tod des Tieres.

Die Vakzine wird in einer Menge verabreicht, die sich in präklinischen bzw. klinischen Versuchen als geeignet herausgestellt hat. Für die Präklinik werden die entsprechenden Tiermodelle herangezogen.

Im weiteren kann der erfindungsgemässe Komplex auch zur Herstellung einer Präparation zur Immunisierung von Säugern oder Vögel verwendet werden. Eine derartige pharmazeutische Präparation enthält den erfindungsgemässen Komplex in einer für die Immunisierung von Säugern und Vögeln geeigneten Menge, sodass eine ausreichende Antikörperbildung gewährleistet ist.

Nach Immunisierung kann weiters eine polyspezifische Immunglobulinpräparation aus einer Körperflüssigkeit des Säugers, z.B. aus Plasma, Serum oder Kolostrum bzw. aus Eiern der Vögel gewonnen werden. Die polyspezifische Immunglobulinpräparation weist mindestens eine Spezifität für das Antigen und eine Spezifität für den Zellbestandteil auf, und ist erhältlich aus einem entsprechend immunisierten Säuger oder Vogel.

Für die Herstellung der Immunglobulinpräparation wird beispielsweise in bestimmten Zeitintervallen das erfindungsgemässe Immunogen an einen Säuger verabreicht, beispielsweise an eine Maus, Ziege oder Kaninchen. Diesem Säuger wird dann eine Körperflüssigkeit, z.B. Blut oder Colostrum, entnommen. Nach gängigen Methoden wird daraus die Immunglobulin-Fraktion isoliert. Die enthaltenen Antikörper sind vorzugsweise gegen eine Mehrzahl protektiver Antigene gerichtet, welche von einem Pathogen in der frühen Phase der Replikation gebildet werden. Nach einer weiteren Ausführungsform können aber auch aufgrund einer gezielten Auswahl der Antigene Antikörper gewonnen werden, die gegen jene protektiven Antigene gerichtet sind, die das Pathogen in der späten Phase der Replikation bildet. Je nach Auswahl der Antigene können in der erfindungsgemässen Immunglobulin-Präparation auch Antikörper gegen prämature Antigene oder nicht-strukturelle Antigene enthalten sein. Dies ist insbesondere bei der passiven Immunisierung mit Antikörpern von Vorteil, da infizierte bzw. mit einem rekombinanten Gen transformierte bzw. transfizierte Wirtszellen von diesen Antikörpern erkannt und eliminiert werden können.

Es kann ferner eine pharmazeutische Präparation auf Basis einer Nukleinsäure und eines Zellbestandteils hergestellt werden, welche Nukleinsäure ein Antigen exprimieren kann, das mit dem Zellbestandteil einen Komplex nach Anspruch 1 bildet. Das Antigen ist von einem Pathogen abgeleitet oder das Pathogen in inaktiver Form. Das Pathogen kann, wie bereits an früherer Stelle erwähnt, ein Virus, ein Bakterium, ein Parasit, aber auch ein molekulares Pathogen, z.B. Prion, sein.

Sowohl die Nukleinsäure als auch der Zellbestandteil können nicht nur in isolierter, sondern auch in gereinigter Form vorliegen. Jedoch kann der Zellbestandteil auch ohne zusätzliche Reinigung vorliegen, wenn z.B. durch Arbeiten unter sterilen Bedingungen dafür Sorge getragen worden ist, daß keine Kontaminationen vorliegen.

Die Nukleinsäure liegt bevorzugterweise als Plasmid mit einem für die Expression der Nukleinsäure geeigneten Promotor vor. Derartige Plasmide können beispielsweise kommerziell erhältliche Plasmide sein. Als Promotoren kommen gängige Promotoren in Frage, beispielsweise ein CMV- oder RSV Promotor. Im allgemeinen hängt die Wahl des Promotors von der verwendeten Zelle bzw. dem zu exprimierenden Antigen ab.

Die Nukleinsäure bzw. das exprimierte Antigen und der Zellbestandteil liegen bevorzugterweise miteinander assoziiert vor. Die Assoziation kann beispielsweise über Lipide oder über einen Träger, vorzugsweise über ein Adjuvans, erfolgen. Die Assoziation kann über kovalente Bindungen erfolgen, aber auch aufgrund unspezifischer Wechselwirkungen, beispielsweise aufgrund elektrostatischer Kräfte oder van der Waals Kräfte.

Des weiteren ist ein Schritt zur Inaktivierung von gegebenenfalls vorhandenen Pathogen, wie Viren, vor oder nach der Komplexbildung vorgesehen.

Mittels des erfindungsgemässen Komplexes kann auch ein Reagens hergestellt werden, welches zum Antikörpernachweis bzw. zur Erkennung einer Infektion eines Säugers mit dem Pathogen geeignet ist. Insbesondere kann mit dem erfindungsgemässen Reagens eine Infektion mit dem Pathogen von einer Impfung gegen das Pathogen unterschieden werden. Matveeva V.A. et al., 1995, Immunol. Lett. 46, 1-4, konnten zeigen, dass Seren von Patienten mit einer Flavivirus-Infektion Antikörper enthielten, welche mit nicht-strukturellen Proteinen des Virus reagierten. Nach Impfung mit dem Ganzvirus-Impfstoff können jedoch nur Antikörper gegen Strukturproteine des Virus entstehen. Die Unterscheidung erfolgt derart, dass man eine Körperflüssigkeit des Säugers gewinnt, diese mit dem gegebenenfalls auf einem Träger immobilisierten Reagens versetzt und durch ein geeignetes Mittel die Immunogen-Antikörper Reaktion, die für eine Infektion mit dem Pathogen indikativ ist, detektiert.

Erfindungsgemäss wird auch ein Set zur Erkennung einer Infektion eines Organismus mit einem Pathogen zur Verfügung gestellt. Dieses Set umfasst den erfindungsgemässen Komplex und ein Mittel zur Detektion der spezifischen Reaktion auf den Kontakt mit dem erfindungsgemässen Komplex, z.B. einer Komplex-Antikörper Reaktion, welche indikativ ist für eine Infektion.

Das Mittel zur Detektion ist beispielsweise ein markiertes Substrat, wie Antikörper, die spezifisch für die zu detektierenden Antikörper, beispielsweise IgM oder IgG, sind. Die Markierung erfolgt gemäss gängiger Methoden, beispielsweise mit einem Chromogen, Fluorogen oder radioaktivem Stoff.

Erfindungsgemäss werden auch Verfahren zur Herstellung des erfindungsgemässen Komplexes zur Verfügung gestellt. Ein erstes Verfahren zur Herstellung des Komplexes umfasst die folgenden Schritte
- Gewinnen eines Antigens aus dem Pathogen oder aus der mit dem Pathogen infizierten Zelle oder durch ein chemisches bzw. biotechnologisches Verfahren,
- Gewinnen des Zellbestandteiles der Säugetierzelle,
- Binden des Antigens an den Zellbestandteil, sodass der Komplex erhalten wird,
- Isolieren des Komplexes und
- gegebenenfalls Reinigen des Komplexes.
   Ein weiteres Verfahren zur Herstellung des erfindungsgemässen Komplexes umfasst die Schritte
- Infizieren einer Säugetierzelle mit dem Pathogen,
- gegebenenfalls Behandeln der Zelle zur Freisetzung einer Zellfraktion,
- Isolieren der Zelle bzw. der Zellfraktion, in der der Komplex enthalten ist, und
- gegebenenfalls Reinigen des Komplexes.
   Noch ein Verfahren zur Herstellung des Komplexes umfasst die Schritte
- Gewinnen eines rekombinanten Gens kodierend für das Antigen,
- Transfizieren bzw. Transformieren einer Säugetierzelle mit dem rekombinanten Gen,
- gegebenenfalls Behandeln der Zelle zur Freisetzung einer Zellfraktion,
- Isolieren der Zelle bzw. der Zellfraktion, in der der Komplex enthalten ist, und
- gegebenenfalls Reinigen des Komplexes.

Gemäß einer bevorzugten Ausführungsform ist die Säugetierzelle bzw. die Zellfraktion an wenigstens einem Bestandteil gemäß einem der Ansprüche 9, 14 oder 15 angereichert.

Der Komplex ist in einer besonders bevorzugten Ausführungsform ein Virus, gegebenenfalls in inaktiver Form, welches mit wenigstens einem der Bestandteile gemäß einem der Ansprüche 9, 14 oder 15 angereichert ist.

Die nachfolgenden Beispiele sollen die Erfindung noch näher erläutern, ohne sie jedoch darauf zu beschränken.

### Beispiel 1

### Komplex auf Basis eines Zellbestandteils virus-infizierter Zellen

### a) Zellen

3T3 Maus-Fibroblasten (gezüchtet von BALB/c Mäusen, Haplotypus H2^{d}) wurden in RPMI (GIBCO BRL, Gaithersburg, MD) mit 5% Hitze-inaktiviertem fötalem Kälberserum (HyClone Laboratories Logan, UT), 2 mM Glutamin, 100 *µ*g/ml Streptomyzin und 100 U/ml Penicillin G (alles von JRH Biosciences, Lenexa, KS) bei 37°C, 96% relativer Luftfeuchtigkeit und 5% CO₂ kultiviert.

### b) Virus

Frühsommer Meningoenzephalitis Virus (FSME-Virus), Stamm Neudörfl, wurde durch Ultrafiltration (100 kD cut-off) und Ultrazentrifugation aus dem Überstand von infizierten Vero Zellen gemäss WO 91/09935 gereinigt. Zur Infektion wurde das Virus entsprechend mit steriler Phosphat-gepufferter, physiologischer Kochsalzlösung (PBS) verdünnt.

### c) Virustitration

Der Titer an infektiösem FSME-Virus wurde durch Titration auf PS Zellen bestimmt, modifiziert nach DE Madrid AT et al., Bull. World Health Organ., 1969, 40, 113-21. Dazu werden konfluente Einzellschichten von PS Zellen für eine Stunde mit seriellen zehnfachen Verdünnungen einer zu titrierenden Proben inkubiert, die Probe anschliessend abgesaugt und durch ein Karboxymethyzellulose-haltiges Medium ersetzt. Nach vier Tagen werden die Zellen mit Formaldehyd fixiert und mit Kristallviolett gefärbt. Gegen einen hellen Hintergrund werden die im Zeilrasen entstandenen Löcher, sog. "plaques" gezählt, wobei jedes Loch einem infektiösen Viruspartikel entspricht. Unter Berücksichtigung des Verdünnungsfaktors erhält man die Konzentration an infektiösen Viruspartikel in der Probe, welche in "plaque forming units" (pfu) pro ml angegeben wird.

### d) Virusinaktivierung, Zellpräparation

Die Zellen wurden mit dem Virus infiziert, das Kulturmedium von den adhärenten Zellen abgesaugt, die Zellen zwei mal mit 37°C PBS gewaschen, und schliesslich für 10 Minuten mit einer Lösung von 4% (w/v) Paraformaldehyd in PBS zur Inaktivierung inkubiert. Die Zellen wurden nochmals gewaschen, mit einem Zellkratzer (Costar) von der Zellkulturflasche geschabt, in PBS suspendiert und zentrifugiert (1400 rpm - 10 Minuten - 4°C). Der Zellniederschlag wurde resuspendiert, die Zellkonzentration in einer Zählkammer bestimmt und die Zellsuspension auf eine gewünschte Konzentration eingestellt.

### e) Immunisierung

Zur Immunisierung wurden die Zellen auf die angegebenen Konzentrationen eingestellt, und zwar entweder in 0,9% Al(OH)₃ oder in PBS. Mit Alum adjuvantierte Zellen wurden für subkutane (sc) Applikation verwendet, nichtadjuvantierte Zellen wurden intraperitoneal (ip) injiziert, jeweils in einem Volumen von 0,2 ml pro Versuchtstier. Als Versuchstier wurden weibliche Innzuchtmäuse von den Stämmen BALB/c (Haplotypus H2^{d}) oder C3H (Haplotypus H2^{k}) mit einem Gewicht von 15-17 g verwendet.

### f) Bestimmung der Immunität

Nach Abschluss der Immunisierungen wurde die Immunität der Tiere gegenüber einer Virusexposition getestet. Dazu wurde immunisierten Tieren und einer unbehandelten Kontrollgruppe jeweils 1000 pfu pro Tier in einem Volumen von 0,2 ml i.p., entsprechend etwa einer 100-fachen Letaldosis für 50% der Tiere (LD₅₀), injiziert.

Die Spezifität der humoralen Immunität für bestimmte Virusantigene wurde auf einem "Western blot" bestimmt. Als Antigenpräparation wurden dabei infizierte oder nicht infizierte 3T3 Fibroblasten in einem Lysepuffer (20 mM Tris, 150 mM NaCl, 1% NP-40 Detergens, pH 7,5 mit den Proteaseinhibitoren Phenyl-methyl-sulfonyl-fluorid [2 mM], Aprotinin und Leupeptin [beide 10 *µ* g/ml] aufgelöst, und der durch Zentrifugation erhaltene klare Überstand, eine komplexe Mischung aus Virus- und Zellproteinen, auf einem Polyacrylamidgel elektrophoretisch aufgetrennt (siehe Laemmli UK, Nature, 1970, 227, 680-5). Die aufgetrennten Proteine wurden weiters elektrophoretisch auf eine Nitrozellulosemembran transferiert (siehe Towbin H, et al., Prof. Natl. Acad. Scie., USA, 1979, 76, 4350-4). Nach Blockieren weiterer Proteinbindungsstellen mit einer Milchpulverlösung (1%) wurde die so erhaltene Membrand weiters mit Seren von immunisierten oder Kontrolltieren inkubiert, gebundene Antikörper durch einen Meerrettichperoxidasegekoppelten Zweitantikörper gegen Maus Immunglobulin gebunden, und diese Bindung mittels "enhanced chemiluminescence" (Amersham) detektiert. Durch Vergleich mit einem Molekulargewichtsmarker wurde die Grösse der detektierten Proteinbanden ermittelt.

### g) Resultate und Diskussion

Die Immunisierung von Mäusen nach der beschriebenen Prozedur führte zu einer protektiven Immunantwort gegen eine nachfolgende Infektion mit einer 100-fachen LD₅₀ Dosis an FSME-Virus. Die Protektion wurde nach dreimaligem Immunisieren der Tiere im Abstand von je 2 Wochen mit einigen 5 x 10⁷ Zellen erreicht. Zwischen Applikation einer subkutanen in Alum oder intraperitoneal ohne Adjuvans war kein bedeutender Unterschied festzustellen.

Bezüglich der Immunisierung von Tieren mit homologen (histoident) oder heterologen (histoinkompatibel) Zellen war interessanterweise festzustellen, dass homologe Immunisierung offensichtlich eine bessere Protektion induziert als heterologe. Obwohl bisher passive Protektion durch Transfer von FSME-Virus Antikörpern (siehe Heinz FX et al., Virology, 1983, 126, 525-37) oder Antiseren (siehe Heinz FX et al., Infect Immun., 1981, 33, 250-7) demonstriert werden konnten, muss man vor dem Hintergrund dieser experimentellen Daten annehmen, dass auch zellmediierte Immunität in dem beschriebenen, experimentellen System einen Beitrag zur Protektion leistet.

Dass bei beiden Immunisierungswegen eine humorale Immunität erreicht wurde, konnte im "Western blot" gezeigt werden, da Seren von beiden Gruppen klare Reaktivität mit Viruskodierten Proteinen aufweisen. Besonders bemerkenswert in diesem Zusammenhang ist weiterhin, dass nicht nur das virale Oberflächenprotein, glyko-E, sondern ebenso auch zumindest ein nicht-strukturelles Protein des Virus erkannt wurde z.B. NS1. dieses Protein wird zwar vom FSME-Virus Genom kodiert, ist aber im reifen Virion nicht vorhanden. Das Protein wird aber bekannterweise auf der Oberfläche von Flavivirus-infizierten Zellen, im speziellen auch mit FSME-Virus infizierten Zellen, exprimiert und Immunisierung mit NS1 von Flaviviren kann eine protektive Immunität. induzieren. Während bei konventionellen Immunisierungen mit einem inaktivierten Virus diese nicht-strukturellen Determinanten dem Immunsystem nicht angeboten werden, kann mit dem hier beschriebenen Weg Immunität sowohl gegen licht-strukturelle als auch gegen strukturelle Proteine eines Virus induziert werden. Auch bei Verabreichung suboptimalern Mengen an FSME-Virus an die Tiere konnte eine klar verlängerte mittlere Überlebenszeit festgestellt werden.

### Beispiel 2

### Komplex auf Basis transfizierter Zellen

Als Immunogen wurden 3T3-Zellen (3T3, H-2D^{d}, ATCC CCL163) oder 3T3-Zellen, die mit dem für gp160 kodierenden Gen des Humanen Immundefizienz Virus Typ I transfiziert worden raren, verwendet (3T3-gp160, hergestellt nach Felgner et al. 1987. Proc. Natl.Acad.Sci., USA, 84, 7413). Vor der Immunisierung wurden die Zellen mit 50 Gy einer ¹³⁷Cs-Quelle bestrahlt. Mit je 5x10⁶ 3T3 oder 3T3-gp160 wurden dann Balb/c Mäuse (Charles River, Sulzfeld, Deutschland) intraperitoneal immunisiert. Diese Immunisierung wurde in dreiwöchigem Abstand noch zweimal wiederholt. Eine Woche nach der letzten Immunisierung wurde dann den Mäusen zur Bestimmung der zellulären Immunität die Milz, sowie zur Bestimmung der humoralen Immunität Blut entnommen.

### a) Zelluläre Immunität

Die Fähigkeit des erfindungsgemässen Komplexes, eine zellulären Immunität zu induzieren, wurde anhand des Nachweises einer T Zell-vermittelten Zytotoxizität bestimmt. Dazu wurde aus der Milz durch vorsichtiges Passieren durch ein Drahtsieb, Filtration durch sterile Baumwollwatte und anschliessende Haemolyse der Erythrozyten eine Einzelzellsuspension der immunkompetenten Zellen gewonnen. Diese Zellen wurden dann in komplettem Kulturmedium resuspendiert, welches aus RPMI 1640 (Flow Laboratories, Irvine, UK) mit dem Zusatz von 10% hitzeinaktiviertem fötalem Kälberserum (JRH Biosciences, Lenexa, KS), 2 mM L-Glutamin (Gibco, Paisley, Scotland), 100 IU/mL Penizillin und 100 ug/ml Streptomycin (beide von Gibco), sowie 5x10⁻⁵ M 2-Mercaptoethanol (Biorad, Hercules, CA, USA) besteht. Zur in vitro Restimulation der Zytotoxischen T-Zellen wurden die immunkompetenten Milzzellen in einer Dichte von 5x10⁶ Zellen pro Kulturnapf einer "24-well" Kulturplatte (Costar) ausgesät und durch Zugabe von 0,15 uM gp120 Peptid 312-327 (Neosystems, Strassburg, Frankreich) stimuliert.

Nach 5-tägiger Inkubation bei 37°C und 5% CO₂ wurden die Milzzellen wieder geerntet und die toten Zellen durch Zentrifugation über einen Lympholyte-M (Cedarlane, Ontario, Cananda) Gradienten entfernt. Die lebenden Milzzellen wurden dann als Effektorzellen zur Bestimmung der zellvermittelten Zytotoxizität in einem ⁵¹Cr Freisetzungstest verwendet. Dazu wurden zunächst als Targetzellen 3T3 und 3T3-gp160 Zellen mit 14.8 MBq Na₂⁵¹CrO₄ (Amersham, Amersham, Buckinghamshire, UK) für 2 Stunden radioaktiv markiert. Jeweils 1x10⁴ dieser Targetzellen wurden dann in je zwei Kulturnäpfe einer 96-well V-Boden Mikrotiterkulturplatte (Nunc, Roskilde, Dänemark) pipettiert und die Effektorzellen in dem angegebenen Verhältnis zugegeben. Die Platten wurden dann für 4 Stunden bei 37°C inkubiert und am Ende der Inkubationszeit wurden aus jedem Kulturnapf 100 ul Überstand entnommen. Die darin enthaltene, durch eine eventuelle Lyse der Targetzellen freigesetzte Radioaktivität, wurde anschliessend in einem y-Strahlungs-Zählgerät (Packard, Merridan, CT) gemessen. Die spontane und maximale ⁵¹Cr-Freisetzung wurde durch Inkubation der Targetzellen mit Kulturmedium allein, bzw. mit einer Lösung von 1% Triton X-100 bestimmt. Der Prozentsatz der spezifischen Lyse wurde durch die Formel berechnet: (cpm Test-Freisetzung - cpm Spontan-Freisetzung)/(cpm Maximal-Freisetzung - cpm Spontan-Freisetzung).

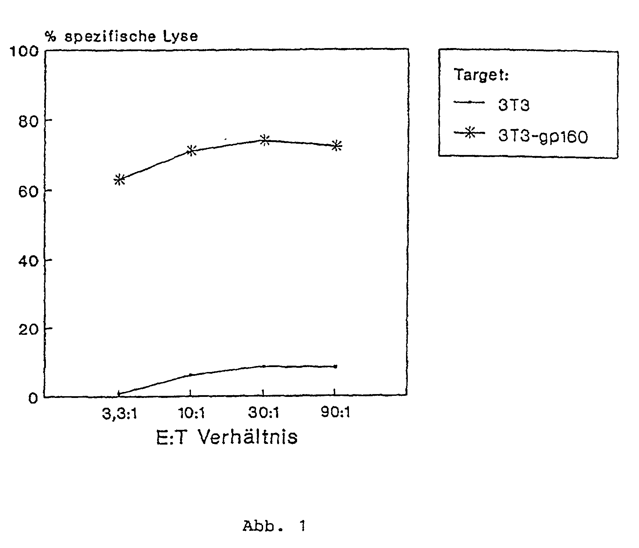

Abbildung 1 zeigt den Prozentsatz der spezifischen Lyse durch Effektorzellen aus Mäusen, die mit gp160 IIIB transfizierten 3T3 immunisiert worden waren. Während diese Effektozellen die transfizierten Targetzellen bis zu 79% lysierten, wurden die nicht transfizierten 3T3 nur geringfügig lysiert.

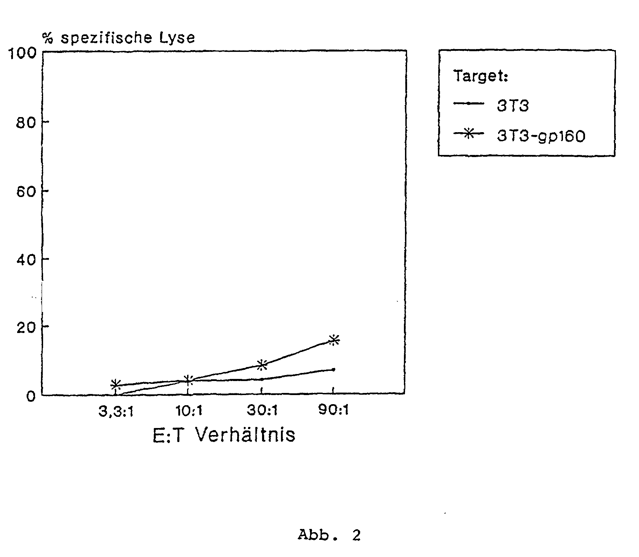

Abbildung 2 zeigt dagegen den Prozentsatz der spezifischen Lyse durch Effektorzellen aus Mäusen, die mit nicht transfizierten 3T3 immunisiert worden waren. Diese Effektozellen erreichten keine nennenswerte Lyse der Targetzellen. Die Ergebnisse beweisen somit, dass das erfindungsgemässe Konstrukt in der Lage ist, eine HIV-1 spezifische zelluläre Immunantwort, insbesondere gegen das HIV-1 Hüllprotein gp160 gerichtete zytotoxische T-Zellen, zu stimulieren.

### b) Humorale Immunität

Zur Bestimmung der humoralen Immunantwort wurde den immunisierten Mäusen unter leichter Ätherbetäubung Blut entnommen. Daraus wurde ein Serum gewonnen, welches mit Hilfe eines Enzymimmunoassays auf IgG-Antikörper gegen gp120 IIIB getestet wurde. Dazu wurden die Kulturnäpfe einer 96 Well-Platte (Flachboden, high binding,FA Costar Cambridge MA USA) mit je 100 ul einer Lösung von gp120 (HIV-1 IIIB, Intracell, London) in einer Konzentraton von 1ug/ml befüllt und für eine Stunde bei 37°C inkubiert. Nach dieser Beschichtung der Kulturnäpfe mit dem gp120 Protein wurde die Platte gewaschen und restliche freie Bindungsstellen in den Kulturnäpfen wurden mit 250 ul 2% BSA (bovines Serumalbumin) in PBS (phosphatgepufferte Kochsalzlösung) abgesättigt. Die zu testenden Serumproben, bzw. ein Kontrollserum, welches gp160-Antikörper positiv ist, wurden in verschiedenen Verdünnungsstufen in 100 ul pro Kulturnapf aufgetragen. Nach einer Inkubation von 16 Stunden bei 37°C wurde die Platte erneut gewaschen. Zum Nachweis der gebundenen gp120-spezifischen Antikörper wurde eine mit Peroxidase markierte Ziege anti-Maus-IgG Antikörperlösung (Accurate Chem., Westbury, NY, USA) in einer Verdünnung von 1:50 000 für eine Stunde zugegeben. Anschliessend wurde die Platte erneut gewaschen und eine OPD-Substratlösung (ortho-Phenylendiamin, 3 mg/ml, Sigma, ST.Louis, MO, USA) zugegeben. Die Enzymreaktion wurde nach 30 Minuten mit einer 2N Schwefelsäurelösung gestoppt und der gebildete Farbstoff mit einem Nunc-ImmunoReader bei 490 nm (Referenzfilter 620 nm) gemessen. Der Titer der untersuchten Probe ergab sich aus dem Reziprokwert der höchsten Probenverdünnung deren optische Dichte einen höheren Wert als OD 0,2 hatte.
In Tabelle 1 ist unter der Überschrift ELISA der Serumtiter an gp160 IIIB spezifischem IgG der wie oben beschrieben immunisierten Mäuse aufgelistet. Die Werte zeigen, dass das erfindungsgemässe Konstrukt in der Lage ist, auch eine humorale Immunantwort zu induzieren. Die Werte unter der Überschrift Zytotoxizität zeigen nochmals die Induktion einer zellulären Immunantwort.

### Beispiel 3

### Komplex auf Basis transfizierter Zellen mit einem Adjuvans

Als Immunogen wurden gp160 IIIB transfizierte 3T3 Zellen wie unter Beispiel 1 beschrieben verwendet, die zusätzlich entweder mit PBS oder 5 ug/ml Cholera Toxin B (CTB, List Biologicals, Campbell, CA, USA) vermischt wurden. Mit je 5x10⁶ dieser Zellen wurden dann Balb/c Mäuse mit Hilfe einer Fütterungsnadel (Nordland, Hamburg, Deutschland) intubiert und das Immunogen direkt in den Magen verabreicht. Zwei Stunden vorher war den Mäusen das Futter entzogen worden und 30 Minuten vor der Immunisierung wurde die Magensäure der Mäuse durch Verabreichung einer 1,5% NaH₂CO₃ Lösung neutralisiert. Die mukosale Immunisierung wurde in einwöchigem Abstand noch zweimal wiederholt. Eine Woche nach der letzten Immunisierung wurde dann zur Bestimmung der zellulären Immunität wie in Beispiel 1 beschrieben verfahren.

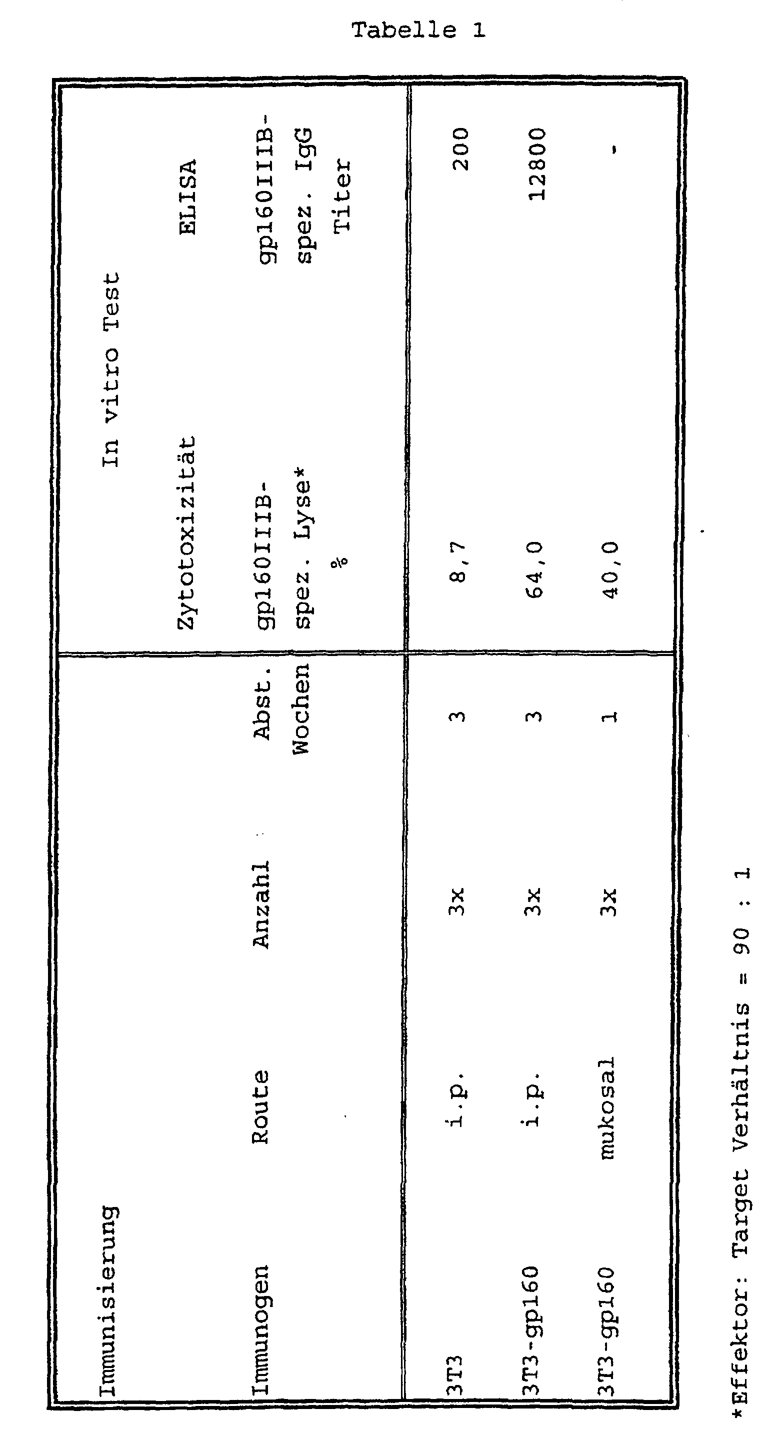

Tabelle 2 zeigt, *dass* auch bei mukosaler Immunisierung mit gp160-transfizierten Zellen (in Gegenwart eines entsprechenden Adjuvans) eine HIV-1 spezifische zellvermittelte Immunität induziert werden konnte.

### Beispiel 4

### Induktion einer Immunantwort durch infizierte Zellen

3T3-Zellen oder Vero-Zellen (ATCC CCL81) wurden mit dem rekombinanten Vaccinia Virus vSC25 (zur Verfügung gestellt von Dr. Barrett, Immuno AG, Orth, Österreich), das die genetische Information für gp160 IIIB enthält, für 16 Stunden mit einer "multiplicity of infection" von 5 infiziert. Mit je 5x10⁶ dieser Zellen oder nicht infizierten Kontrollzellen wurden dann Balb/c Mäuse mit Hilfe einer Fütterungsnadel mukosal immunisiert. Diese Immunisierung wurde in dreiwöchigem Abstand noch zweimal wiederholt. Eine Woche nach der letzten Immunisierung wurde dann zur Bestimmung der zellulären Immunität wie in Beispiel 1 beschrieben verfahren. Tabelle 2 zeigt, dass auch eine mukosale Immunisierung mit rekombinantem Vaccinia Virus infizierten Zellen eine HIV-1 spezifische zytotoxische T-Zell Antwort induzieren konnte. Diese Immunreaktion war unabhängig von der zur Immunisierung verwendeten Zellart.

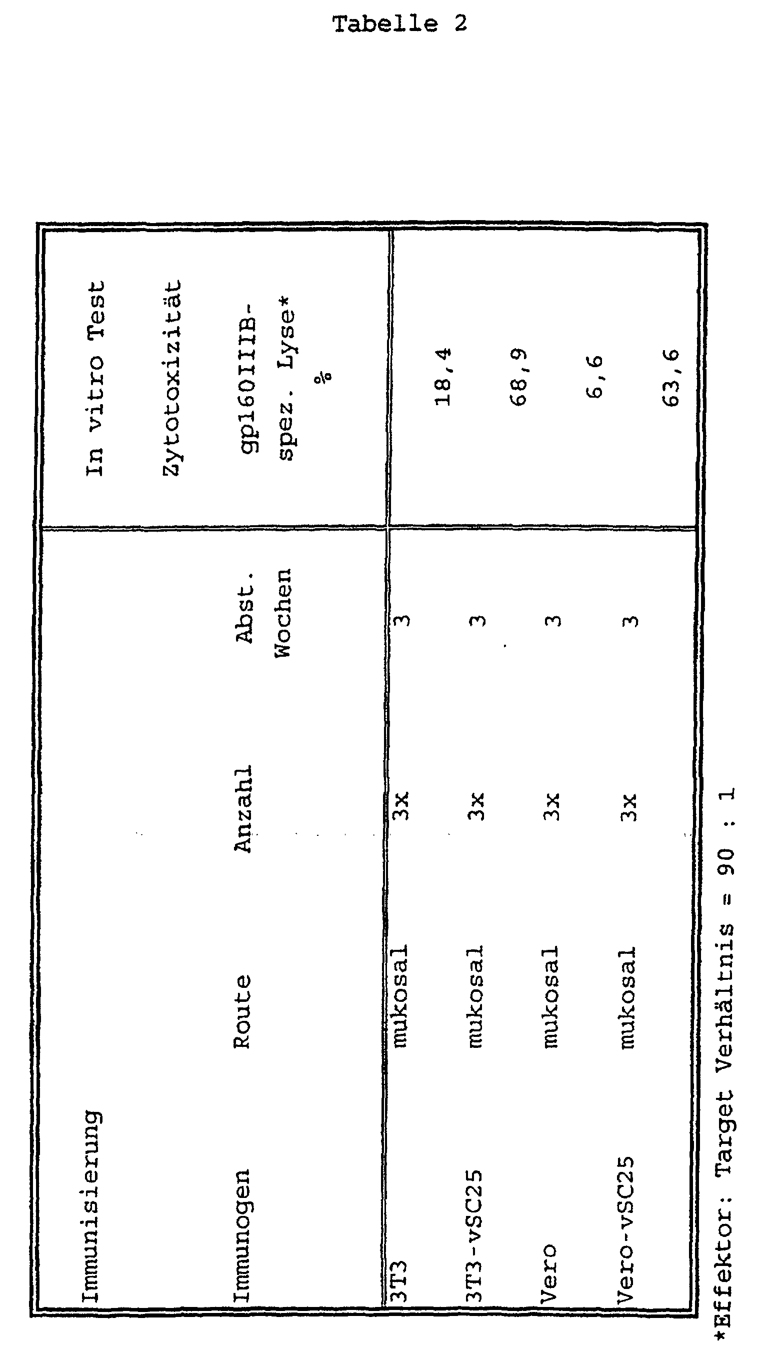

## Patentansprüche

1. Komplex zwischen einem Antigen eines mikrobiellen oder molekularen Pathogens mit Zellbestandteilen von Säugetierzellen in isolierter bzw. gereinigter Form, wobei der Komplex bzw. das Antigen und die Zellbestandteile einer Behandlung zur Pathogen-Inaktivierung unterzogen sind.

2. Komplex nach Anspruch 1, **dadurch gekennzeichnet, dass** das Antigen von einem Virus, Bakterium, Parasiten oder einem Prion abgeleitet ist.

3. Komplex nach Anspruch 1 oder 2, **dadurch gekennzeichnet, daß** das Antigen ein inaktives Virus ist.

4. Komplex nach Anspruch 3, **dadurch gekennzeichnet, daß** das Virus ein inaktiviertes Virus ist und insbesondere ein durch ein chemisches und/oder physikalisches Verfahren inaktiviertes Virus.

5. Komplex nach Anspruch 1 bis 4, **dadurch gekennzeichnet, dass** das Antigen von einer rekombinanten Nukleinsäure kodiert wird.

6. Komplex nach einem der Ansprüche 1 bis 5, **dadurch gekennzeichnet, dass** der Zellbestandteil von infizierten Zellen abgeleitet ist.

7. Komplex nach einem der Ansprüche 1 bis 5, **dadurch gekennzeichnet, dass** der Zellbestandteil von einer ein rekombinantes Gen enthaltenden Zelle abgeleitet ist.

8. Komplex nach einem der Ansprüche 1 bis 5, **dadurch gekennzeichnet, dass** der Zellbestandteil von einer aktivierten Zelle abgeleitet ist.

9. Komplex nach Anspruch 1 bis 5, **dadurch gekennzeichnet, daß** der Zellbestandteil abgeleitet ist von Zellen, die von der Spezies stammen, für die das Virus pathogen ist.

10. Komplex nach einem der Ansprüche 1 bis 9, **dadurch gekennzeichnet, dass** der Zellbestandteil in einer Fraktion enthalten ist, die die Zellmembran bzw. eine intrazelluläre Struktur der Zelle enthält.

11. Komplex nach einem der Ansprüche 1 bis 10, **dadurch gekennzeichnet, daß** das Virus ein human-pathogenes Virus ist und der Zellbestandteil abgeleitet ist von humanen Zellen.

12. Komplex nach einem der Ansprüche 1 bis 11, **dadurch gekennzeichnet, dass** die Komplexbindung eine kovalente Bindung darstellt, gegebenenfalls durch ein Bindeglied ("linker").

13. Komplex nach einem der Ansprüche 1 bis 12, **dadurch gekennzeichnet, dass** die Komplexbindung aufgrund von elektrostatischen, hydrophoben oder van der Waals schen Kräften besteht.

14. Komplex nach einem der Ansprüche 1 bis 13, **dadurch gekennzeichnet, daß** in der Zellfraktion eine Substanz ausgewählt aus der Gruppe Membranprotein, intrazelluläres Protein, von aktivierten Lymphozyten abgeleiteter Faktor und Aktivator für Lymphozyten enthalten ist oder eine Kombination davon.

15. Komplex nach einem der Ansprüche 1 bis 14, **dadurch gekennzeichnet, daß** das Membranprotein und/oder das intrazelluläre Protein ein Alloantigen, Chemokin-Rezeptor, Chemokin oder Lymphokin ist oder eine Kombination davon.

16. Komplex nach einem der Ansprüche 1 bis 15, **dadurch gekennzeichnet, daß** der Komplex aus einem inaktiven Virus nach Anspruch 3 besteht, welches einen Zellbestandteil gemäß einem der Ansprüche 9, 14 oder 15 inkorporiert hat.

17. Komplex nach einem der Ansprüche 1 bis 16, **dadurch gekennzeichnet, dass** das Antigen und der Zellbestandteil an einem festen Träger, insbesondere an einer Lipidkomponente, adsorbiert sind.

18. Komplex nach einem der Ansprüche 1 bis 17, **dadurch gekennzeichnet, dass** er aus einer mit dem Pathogen infizierten Zelle erhältlich ist.

19. Komplex nach einem der Ansprüche 1 bis 18, **dadurch gekennzeichnet, dass** er aus einer ein rekombinantes Gen enthaltenden Zelle erhältlich ist.

20. Impfstoffzusammensetzung enthaltend den Komplex nach einem der Ansprüche 1 bis 19, in einer geeigneten Menge für die prophylaktische oder therapeutische Behandlung von Säugern, insbesondere Primaten, gegen die Erkrankung mit dem Pathogen, in einer pharmazeutisch akzeptablen Form.

21. Imgfstoffzusammensetzung nach Anspruch 20, **dadurch gekennzeichnet, dass** sie weitere Antigene bzw. Adjuvantien enthält.

22. Pharmazeutische Präparation enthaltend den Komplex nach einem der Ansprüche 1 bis 19, in einer zur Immunisierung von Säugern oder Vögeln geeigneten Menge.

23. Verfahren zur Herstellung des Komplexes nach einem der Ansprüche 1 bis 19, umfassend die folgenden Schritte
• Gewinnen eines Antigens aus dem Pathogen oder aus der mit dem Pathogen infizierten Zelle oder durch ein chemisches bzw. biotechnologisches Verfahren,
• Gewinnen des Zellbestandteiles der Säugetierzelle,
• Binden des Antigens an den Zellbestandteil, sodass der Komplex erhalten wird,
• Isolieren des Komplexes und
• gegebenenfalls Reinigen des Komplexes,
wobei der Komplex bzw. das Antigen und der Zellbestandteil einer Behandlung zur Pathogen-Inaktivierung unterzogen werden.

24. Verfahren zur Herstellung des Komplexes nach einem der Ansprüche 1 bis 19 umfassend die folgenden Schritte
• Infizieren einer Säugetierzelle mit dem Pathogen,
• Behandeln der Zelle zur Freisetzung einer Zellfraktion,
• Isolieren der Zellfraktion, in der der Komplex enthalten ist, und
• gegebenenfalls Reinigen des Komplexes, wobei der Komplex einer Behandlung zur Pathogen-Inaktivierung unterzogen wird.

25. Verfahren zur Herstellung des Komplexes nach einem der Ansprüche 1 bis 19 umfassend die folgenden Schritte
• Gewinnen eines rekombinanten Gens kodierend für das ' Antigen,
• Transfizieren bzw. Transformieren einer Säugetierzelle mit dem rekombinanten Gen,
• Behandeln der Zelle zur Freisetzung einer Zellfraktion,
• Isolieren der Zellfraktion, in der der Komplex enthalten ist, und
• gegebenenfalls Reinigen des Komplexes,
wobei der Komplex einer Behandlung zur Pathogen-Inaktivierung unterzogen wird.

26. Verfahren nach einem der Ansprüche 23 bis 25, **dadurch gekennzeichnet, daß** die Zellfraktion an wenigstens einem Bestandteil gemäß einem der Ansprüche 9, 14 oder 15 angereichert ist.

27. Verfahren nach Anspruch 24 oder 26, **dadurch gekennzeichnet, daß** der Komplex ein Virus, gegebenenfalls in inaktiver Form, ist, welches mit wenigstens einem der Bestandteile gemäß einem der Ansprüche 9, 14 oder 15 angereichert ist.

## Claims

1. A complex between an antigen of a microbial or molecular pathogen with cell constituents of mammalian cells in isolated and/or purified form, the complex or the antigen and the cell constituents being subjected to a treatment for pathogen inactivation.

2. A complex according to claim 1, **characterized in that** the antigen is derived from a virus, bacterium, parasite or a prion.

3. A complex according to claim 1 or 2, characterized that the antigen is an inactive virus.

4. A complex according to claim 3, **characterized in that** the virus is an inactivated virus and, in particular, a virus inactivated by a chemical and/or physical process.

5. A complex according to claims 1 to 4, **characterized in that** the antigen is encoded by a recombinant nucleic acid.

6. A complex according to anyone of claims 1 to 5, **characterized in that** the cell constituent is derived from infected cells.

7. A complex according to anyone of claims 1 to 5, **characterized in that** the cell constituent is derived from a cell containing a recombinant gene.

8. A complex according to anyone of claims 1 to 5, **characterized in that** the cell constituent is derived from an activated cell.

9. A complex according to claims 1 to 5, **characterized in that** the cell constituent is derived from cells originating from the species for which the virus is pathogenic.

10. A complex according to anyone of claims 1 to 9, **characterized in that** the cell constituent is contained in a fraction containing the cell membrane or an intracellular structure of the cell.

11. A complex according to anyone of claims 1 to 10, **characterized in that** the virus is a human pathogenic virus and the cell constituent is derived from human cells.

12. A complex according to anyone of claims 1 to 11, **characterized in that** the complex linkage is a covalent linkage, optionally by means of a linker.

13. A complex according to anyone of claims 1 to 12, **characterized in that** the complex linkage is effected by electrostatic, hydrophobic or van der Waals' forces.

14. A complex according to anyone of claims 1 to 13, **characterized in that** a substance selected from the group consisting of membrane protein, intracellular protein, factor derived from activated lymphocytes and activator for lymphocytes or a combination thereof is contained in the cell fraction.

15. A complex according to anyone of claims 1 to 14, **characterized in that** the membrane protein and/or the intracellular protein is an alloantigen, chemokine receptor, chemokine or lymphokine or is a combination thereof.

16. A complex according to anyone of claims 1 to 15, **characterized in that** the complex is comprised of an inactive virus according to claim 3, which has a cell constituent according to anyone of claims 9, 14 or 15 incorporated.

17. A complex according to anyone of claims 1 to 16, **characterized in that** the antigen and the cell constituent are adsorbed on a solid carrier, in particular a lipid component.

18. A complex according to anyone of claims 1 to 17, **characterized in that** the complex is obtainable from a cell infected with the pathogen.

19. A complex according to anyone of claims 1 to 18, **characterized in that** the complex is obtainable from a cell containing a recombinant gene.

20. A vaccine composition containing the complex according to anyone of claims 1 to 19, in an amount suitable for the prophylactic or therapeutic treatment of mammals, in particular primates, against disease caused by the pathogen, in a pharmaceutically acceptable form.

21. A vaccine composition according to claim 20, **characterized in that** the vaccine composition contains additional antigens or adjuvants.

22. A pharmaceutical preparation containing the complex according to anyone of claims I to 19, in an amount suitable for the immunization of mammals or birds.

23. A process for the preparation of the complex according to anyone of claims 1 to 19, comprising the following steps:
• obtaining an antigen from the pathogen or from the cell infected with the pathogen or by a chemical or biotechnological method,
• obtaining the cell constituent of the mammalian cell,
• linking the antigen to the cell constituent so as to obtain the complex,
• isolating the complex and,
• optionally, purifying the complex,
the complex or the antigen and the cell constituent being subjected to a treatment for pathogen inactivation.

24. A process for the preparation of the complex according to anyone of claims 1 to 19, comprising the following steps:
• infecting a mammalian cell with the pathogen,
• treating the cell in order to release a cell fraction,
• isolating the cell fraction in which the complex is contained and,
• optionally, purifying the complex,
the complex being subjected to a treatment for pathogen inactivation.

25. A process for the preparation of the complex according to anyone of claims 1 to 19, comprising the following steps:
• obtaining a recombinant gene coding for the antigen,
• transfecting or transforming a mammalian cell with the recombinant gene,
• treating the cell in order to release a cell fraction,
• isolating the cell fraction in which the complex is contained and,
• optionally, purifying the complex,
the complex being subjected to a treatment for pathogen inactivation.

26. A process according to anyone of claims 23 to 25, **characterized in that** the cell fraction is enriched with at least one of the constituents according to anyone of claims 9, 14 or 15.

27. A process according to claim 24 or 26, **characterized in that** the complex is a virus, optionally in the inactive form, which is enriched with at least one of the constituents according to anyone of claims 9, 14 or 15.

## Revendications

1. Complexe entre un antigène d'un agent pathogène microbien ou moléculaire et des constituants cellulaires de cellules de mammifère sous forme isolée ou purifiée, où le complexe ou l'antigène et les constituants cellulaires sont soumis à un traitement pour inactiver l'agent pathogène.

2. Complexe selon la revendication 1, **caractérisé en ce que** l'antigène est issu d'un virus, d'une bactérie, de parasites ou d'un prion.

3. Complexe selon la revendication 1 ou 2, **caractérisé en ce que** l'antigène est un virus inactivé.

4. Complexe selon la revendication 3, **caractérisé en ce que** le virus est un virus inactivé et en particulier un virus inactivé par un procédé chimique et/ou physique.

5. Complexe selon les revendications 1 à 4, **caractérisé en ce que** l'antigène est codé par un acide nucléique recombiné.

6. Complexe selon l'une des revendications 1 à 5, **caractérisé en ce que** le constituant cellulaire est issu de cellules infectées.

7. Complexe selon l'une des revendications I à 5, **caractérisé en ce que** le constituant cellulaire est issu d'une cellule contenant un gène recombiné.

8. Complexe selon l'une des revendications 1 à 5, **caractérisé en ce que** le constituant cellulaire est issu d'une cellule activée.

9. Complexe selon les revendications 1 à 5, **caractérisé en ce que** le constituant cellulaire est issu de cellules qui proviennent de l'espèce pour laquelle le virus est pathogène.

10. Complexe selon l'une des revendications 1 à 9, **caractérisé en ce que** le constituant cellulaire est contenu dans une fraction qui contient la membrane cellulaire ou une structure intracellulaire de la cellule.

11. Complexe selon l'une des revendications 1 à 10, **caractérisé en ce que** le virus est un virus pathogène pour l'homme et le constituant cellulaire est issu de cellules humaines.

12. Complexe selon l'une des revendications 1 à 11, **caractérisé en ce que** la liaison du complexe constitue une liaison covalente, éventuellement par un membre de liaison ("linker").

13. Complexe selon l'une des revendications 1 à 12, **caractérisé en ce que** la liaison du complexe est due à des forces électrostatiques, hydrophobes ou de van der Waals.

14. Complexe selon l'une des revendications 1 à 13, **caractérisé en ce qu'**une substance choisie dans le groupe des protéines membranaires, des protéines intracellulaires, des facteurs issus de lymphocytes activés et des activateurs des lymphocytes, ou une combinaison de ceux-ci, est contenue dans la fraction cellulaire.

15. Complexe selon l'une des revendications 1 à 14, **caractérisé en ce que** les protéines membranaires et/ou les protéines intracellulaires sont un alloantigène, un récepteur de chimiokines, une chimiokine ou une lymphokine, ou une combinaison de ceux-ci.

16. Complexe selon l'une des revendications 1 à 15, **caractérisé en ce que** le complexe consiste en un virus inactivé selon la revendication 3 qui a incorporé un constituant cellulaire selon l'une des revendications 9, 14 et 15.

17. Complexe selon l'une des revendications 1 à 16, **caractérisé en ce que** l'antigène et le constituant cellulaire sont adsorbés sur un support solide, en particulier sur un composant lipidique.

18. Complexe selon l'une des revendications 1 à 17, **caractérisé en ce qu'**il peut être obtenu à partir d'une cellule infectée avec l'agent pathogène.

19. Complexe selon l'une des revendications 1 à 18, **caractérisé en ce qu'**il peut être obtenu à partir d'une cellule contenant un gène recombiné.

20. Composition de vaccin contenant le complexe selon l'une des revendications 1 à 19 en une quantité appropriée pour le traitement prophylactique ou thérapeutique de mammifères, en particulier de primates, contre la maladie avec l'agent pathogène, sous une forme pharmaceutiquement acceptable.

21. Composition de vaccin selon la revendication 20, **caractérisée en ce qu'**elle contient d'autres antigènes ou adjuvants.

22. Préparation pharmaceutique contenant le complexe selon l'une des revendications 1 à 19 en une quantité appropriée pour l'immunisation de mammifères ou d'oiseaux.

23. Procédé de production du complexe selon l'une des revendications 1 à 19 comportant les étapes suivantes :
• obtention d'un antigène à partir de l'agent pathogène ou de la cellule infectée avec l'agent pathogène, ou par un procédé chimique ou biotechnologique,
• obtention du constituant cellulaire de la cellule de mammifère,
• liaison de l'antigène au constituant cellulaire, de sorte que le complexe est obtenu,
• isolement du complexe et
• éventuellement, purification du complexe,
où le complexe ou l'antigène et le constituant cellulaire sont soumis à un traitement pour inactiver l'agent pathogène.

24. Procédé de production du complexe selon l'une des revendications 1 à 19 comportant les étapes suivantes
• infection d'une cellule de mammifère avec l'agent pathogène,
• traitement de la cellule pour libérer une fraction cellulaire,
• isolement de la fraction cellulaire dans laquelle est contenu le complexe, et
• éventuellement, purification du complexe,
où le complexe est soumis à un traitement pour inactiver l'agent pathogène.

25. Procédé de production du complexe selon l'une des revendications 1 à 19 comportant les étapes suivantes :
• obtention d'un gène recombiné codant l'antigène,
• transfection ou transformation d'une cellule de mammifère avec le gène recombiné,
• traitement de la cellule pour libérer une fraction cellulaire,
• isolement de la fraction cellulaire dans laquelle est contenu le complexe, et
• éventuellement, purification du complexe,
où le complexe est soumis à un traitement pour inactiver l'agent pathogène.

26. Procédé selon l'une des revendications 23 à 25, **caractérisé en ce que** la fraction cellulaire est enrichie en au moins un constituant selon l'une des revendications 9, 14 et 15.

27. Procédé selon la revendication 24 ou 26, **caractérisé en ce que** le complexe est un virus, éventuellement sous forme inactivée, qui est enrichi en au moins l'un des constituants selon l'une des revendications 9, 14 et 15.
